# EUROPEAN PATENT APPLICATION

(11) **EP 1 970 378 A1**
(43) Date of publication of application: **17.09.2008**
(21) Application number: 07104297.2
(22) Date of filing: 16.03.2007
(51) Int. Cl.: C07H 23/00, A61K 31/7135, A61P 35/00

(54) **Photoreactive Ru (II) complexes anchored on oligonucleotides, method for obtaining them and use thereof**

(71) Applicant: UNIVERSITE LIBRE DE BRUXELLES, 1050 Brussels (BE); UNIVERSITE JOSEPH FOURIER, F-38041 Grenoble Cédex (FR)
(72) Inventor: Defrancq, Eric, F-38830, SAINT PIERRE D'ALLEVARD (FR); Moucheron, Cécile, B-1160, BRUXELLES (BE); Rickling, Stéphane, F-68740, HIRTZFELDEN (FR); De Mesmaeker (Kirsch), Andrée, B-1050, BRUXELLES (BE)
(74) Representative: pronovem

(57) **Abstract**

The present invention is related to photoreactive Ru (II) complexes anchored on a G-containing oligonucleotide (Formula I), the method for obtaining them and their use, especially for targeting specific nucleotide sequences, to avoid their expression in prokariote or eukaryote cells (especially in bacterial cells, plant cells, animal cells, fungi cells, including yeast cells).

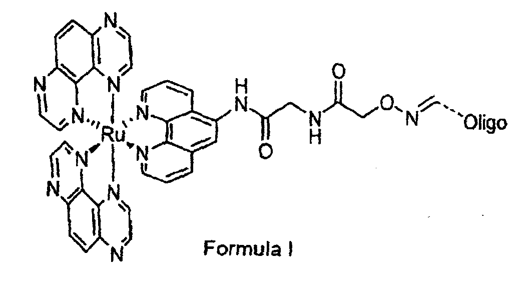

## Description

### Field of the invention

The present invention is related to photoreactive Ru (II) complexes anchored on G-containing oligonucleotides, the method for obtaining them and their use, especially for targeting specific nucleotide sequences, to avoid their expression in prokariote or eukaryote cells (especially in bacterial cells, plant cells, animal cells, fungi cells, including yeast cells).

A preferred use of these oligonucleotides is in the field of anti-tumoral therapy and possibly prevention.

### Background of the invention

Ru(II) complexes containing at least two Tap (1,4,5,8-tetraazaphenanthrene) ligands are known to be oxidant enough in their excited state to photo-oxidize a guanine unit of DNA (1); such a ruthenium complex anchored on one strand of a hybridized oligonucleotide was able to react under illumination with a guanine situated on the complementary strand, which leads to a photocrosslinking of both strands (2) that may be used in anticancer therapy (antisense strategy).

### Aims of the invention

The present invention aims to provide oligonucleotides which are able to lead to photocrosslinking with complementary nucleotide sequences to be targeted and inactivated, and which do not present the drawbacks of the state of the art.

A preferred aim of the present invention is to obtain these oligonucleotides which present a high specificity and are photo-inactivated, if no binding (hybridisation) with their corresponding specific nucleotide sequences to be targeted and inactivated has been obtained.

A further aim of the present invention is to propose such products which will reduce the possible side effects of oligonucleotides which are not bound (hybridized) to their corresponding nucleotide sequences, for instance by avoiding any possible binding (hybridization) upon anti-oncogenic sequences, if no binding has been obtained upon oncogenic sequences.

### Summary of the invention

The present invention is related to photoreactive Ru (II) complexes anchored to oligonucleotides (defined also hereafter as Oligo), wherein these oligonucleotides are guanine-containing oligonucleotides, which means that these oligonucleotides comprise one or more guanine unit(s) (G base)and the method for obtaining them.

These oligonucleotides are preferably antisense or RNAi oligonucleotides sequences (for gene silencing) and made of at least 8, 9, 10, 11, 12, 13, 14, 15, 20, 25, 30, 35, but less than 200, 150, 100, 50, 40 or 35 bases with a photoreactive Ru (II) complex on the 5' or 3' end.

The present invention concerns also this oligo or a method for obtaining this oligo wherein the binding between the photoreactive complex and the oligonucleotides is preferably obtained with a chemical linker, having a suitable end for allowing the binding to the complex.

The present invention is also related to a pharmaceutical composition or a diagnostic kit comprising these elements (photoreactive Ru (II) complex anchored on these oligonucleotides) and possibly a photon (hv) or a device that emits photons.
The pharmaceutical composition may also comprise an adequate pharmaceutical carrier or diluent. In the diagnostic kit according to the invention, one may also include a device that emits photons,

Another aspect of the present invention is related to the use of this photoreactive Ru (II) complex anchored on oligonucleotides combined with a photon for use as a medicament.

A further aspect of the present invention is related to the use of the pharmaceutical composition for the manufacture of a medicament in the treatment of a disease induced by expression of specific nucleotide sequences such as oncogenes whose expression should be modulated, preferably suppressed.

The adequate pharmaceutical carrier of the composition according to the invention or diluent is selected by the person skilled in the art according to the type of administration required upon specific cells or tissues.

The ratio between the amount of the adequate pharmaceutical carrier or diluent and the active compound is defined by the person skilled in the art, according to the administrated doses to be applied to the mammal subject, including a human patient, the surface of the epithelium to be treated and the possible side effects of the active compound upon the mammal subject, including a human patient.

Preferably, this adequate pharmaceutical carrier is present in a solid or liquid form and these oligonucleotides are possibly incorporated into suitable vectors (cationic vesicles, plasmid, virus, etc) to transform (transfect) the cells.

Preferably, this pathology is an hyper-proliferative pathology (cancer) present in specific cells (tumour cells).

This hyper-proliferative disorder (cancer) treated by the composition of the present invention is a hyper-proliferative disorder affecting epithelial cells, epithelial tumoural cells of the skin, of the digestive track (in particular tumours of rectum, colon, intestine, stomach, oesophagus, pharynx, or buccal cavity), tumoural cells of the lung, or tumoural cells affecting a genital or an urinary organ, in particular, the ureter, the urethra, the prostate, the urinary bladder or the uterus neck) or any other tissue that can be treated by the compound of the invention.

The treatment according to the invention comprises the steps of putting into contact the oligonucleotides according to the invention with the cells to be treated and submitting these cells to an adequate light irradiation (photon emission). Preferably, this light irradiation is obtained by any suitable device that emits photons upon the area of the epithelium to be treated.

Preferably, this device emits light in the visible range for a sufficient time for obtaining an efficient treatment of the tumoural cell.

Advantageously, the photoreactive Ru (II) complexes anchored on G-containing oligonucleotides and this device could be present in a kit of parts, possibly comprising an endoscope or any medical device which could be used for obtaining a contact between an epithelium comprising said tumoural cell and the active compound of the pharmaceutical composition according to the invention under light irradiation.

The present invention will be described in more details in the following non-limited examples, in reference to the enclosed figures.

### Short description of the figures

The figures 1 to 3 show different Ru (II) oligonucleotides according to the invention.

The figure 4 represents a polyacrylamide gel electrophoresis of the different oligonucleotides according to the invention. Denaturating PAGE experiments after 0, 5, 10, 15 and 30 min of illumination:
line 1 to 5: W1T* ss
line 6 to 10: W1T* hybridized with W3 (WIT ds)
line 11 to 15: W1T* and SC1
line 16 to 20: W1T* and SC2

### Detailed description of the invention

A photoactive Ru(II) complex anchored on an oligonucleotide containing a guanine unit is used for the specific recognition of an ODN sequence and the formation, under visible light irradiation, of a photocrosslinking between this ODN and its ruthenium-containing complementary strand. The presence of a guanine base in the ruthenium-labeled oligonucleotide sequence implies an intramolecular photoreaction of this Ru-ODN in the absence of the target sequence, even if it is in presence of any other G containing ODN. This work is the basis of a new type of intelligent drugs called "seppuku molecules", which autodestruct if they do not manage to find their target, leading to a totally non-reactive and non-toxic species.

Three different Ru(II) complexes anchored on a 14-mer oligonucleotide, either in position 3' or 5'were obtained. The complexes used are Ru(Tap)₂Phen²⁺ (which is photoreactive with the guanine) and Ru(Bpy)₂Phen²⁺ and Ru (Phen)₂Phen²⁺ (wich are not photooxydant enough to react with a G base, under illumination) [Bpy = 2,2' bipyridine, Phen = 1,10-phenanthroline, Phen" = 5-((N-(ter-butoxycarbonyl)-O-(carboxymethyl)hydroxylamine)glycinamido)-1,10-phenanthroline]. Ru(Tap)₂Phen²⁺ and Ru(Phen)₂Phen²⁺ are chemically bound to the 3' or 5' end of the ODN via an oxime bond. Ru(Bpy)₂Phen²⁺ is obtained by reacting Ru(Bpy)₂Cl₂ with the derivatized ligand Phen" and is anchored to the oligonucleotides derivatized with an aldehyde formation.

The single stranded and duplex solutions used for the spectroscopic studies were prepared at a concentration of 1.10⁻⁵ M in an aqueous buffer (Tris-HCl 10 mM, NaCl 150 mM, pH = 7). All the measurements have been realised in 600 µL quartz cells (UV select, 1.0 x 0.2 cm). Illuminations have been performed with a Thermo Oriel Xe lamp (500W) (Fairlight, The Netherlands) with a H₂O and aqueous KNO₂ filters. Absorption and emission spectra have been performed on a Perkin-Elmer Lambda 40 UV-Vis spectrometer and a Shimadzu RF-5001PC spectrofluorimeter equipped with a Hamamatsu R928 red-sensitive photomultiplier tube respectively.

Radiolabelling in 5' position of the ODNs have been realised by treating with T4 polynucleotide kinase and [β³²P] ATP at 37°C for 30 min. Hybridization, when necessary, has been performed by incubating the labeled ODN with its complementary strand at 85°C for 5 min and at room temperature for at least 6 hrs. Illuminations for the PAGE experiments have been performed with a He/Cd laser (442 nm) (Melles Griot).

Polyacrylamide gel electrophoresis have been performed through a denaturing (urea 7 M) 20 % polyacrylamide (19:1 ratio of acrylamide to bisacrylamide) with TBE (90 mM Tris-borate, pH = 8, 2 mM EDTA) gel. DNA fragments were visualised by autoradiography with a Storage Phosphor Screen (Amersham) film and were counted with a Phosphor-Imager Storm 860 instrument.

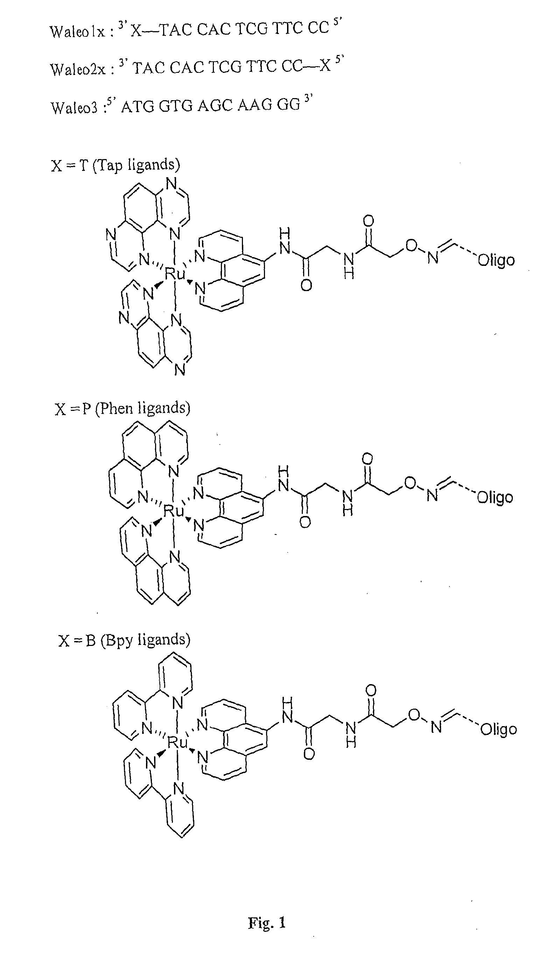

The figure 1 shows the different Ru-oligonucleotides that have been synthesized. These Ru-ODNs have been called Waleo1 if the anchoring of the complex was done on the 3' end and Waleo2 if the anchoring was done on the 5' end. Moreover, if the complex anchored contains Tap ligands, T to the former name was added, if it contains Bpy ligands, a B was added and if it contains Phen ligands, a P was added, so to obtain 6 different Ru-ODNs : Waleo1T, Waleo2T, Waleo1B, Waleo2B, Waleo1P and Waleo2P; Waleo3 is their complementary strand. The important fact to notice is that the oligonucleotide used to support the Ru(II) complex contains a guanine unit which precisely is the base that is oxidized by the photo-oxidizing Ru(II) complexes, if the target complementary strand is not found.

The solutions of the different Ru-ODNs were first illuminated in an aqueous buffer and one may observe the evolution of the absorption and emission spectra as a function of the illumination time. 1.10⁻⁵ M solutions of single stranded and double stranded Waleo1x and Waleo2x (x = T, B or P) were prepared. Illumination of 500 µL of each solution was undertaken and absorption and emission spectra were recorded after 5, 15, 30 and 60 min of illumination. Typical spectra of Waleo1Pss, Waleo1Pds, Waleo1Tss and Waleo1Tds can be seen on the figures.

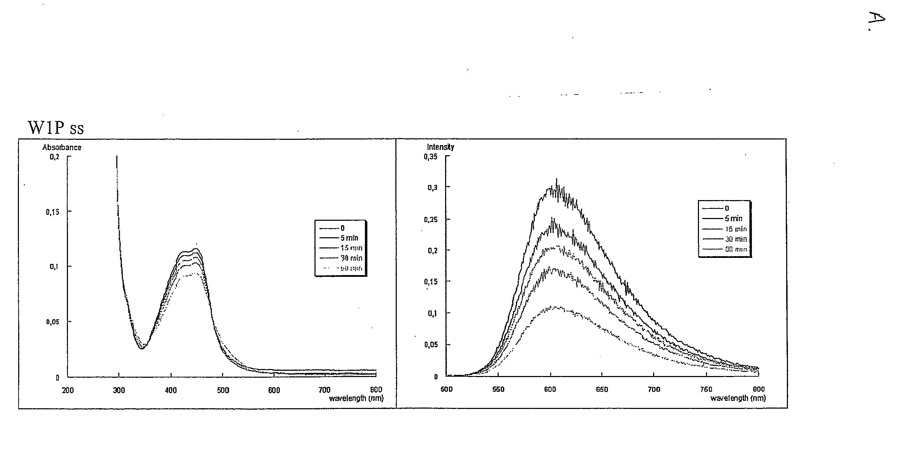

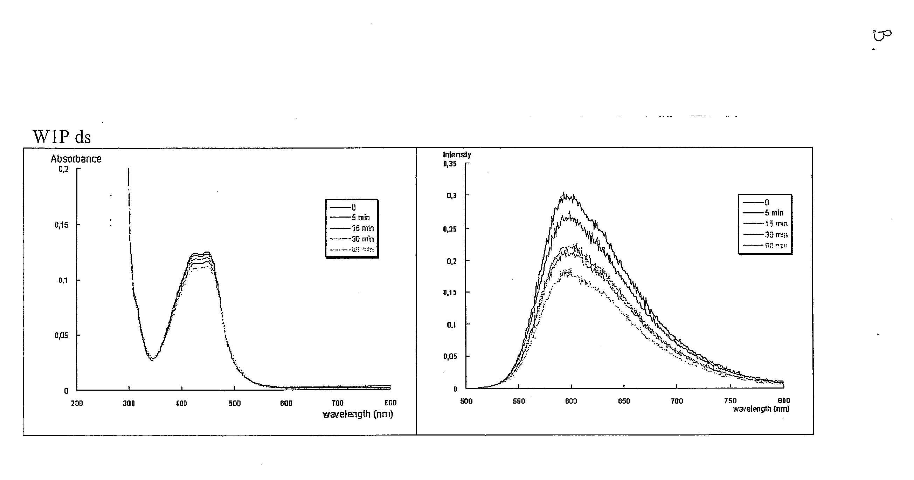

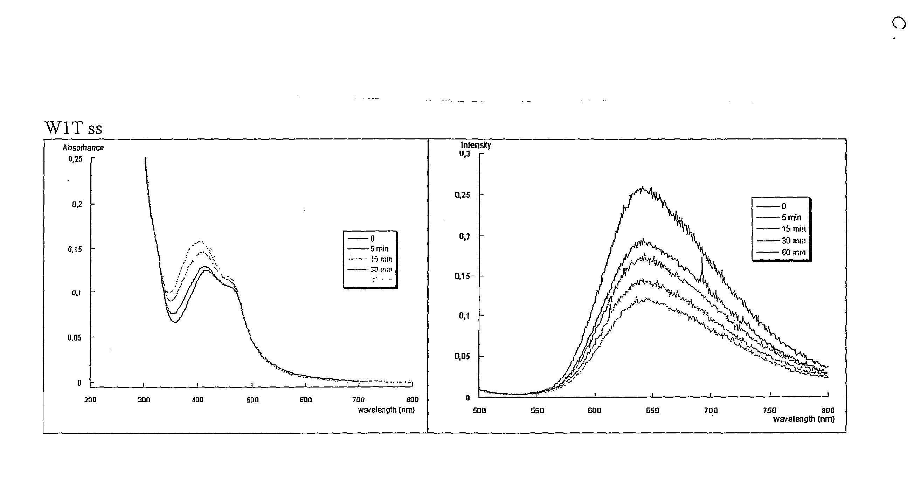

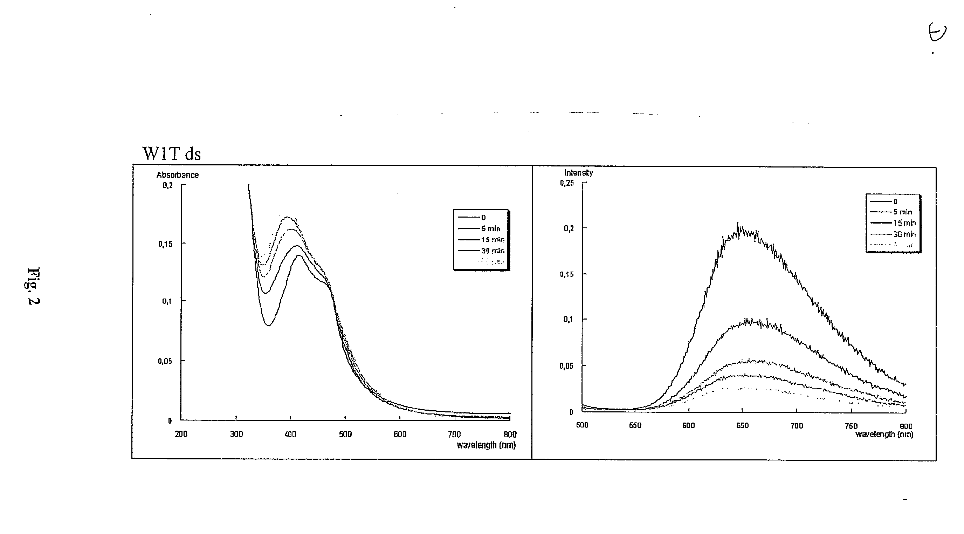

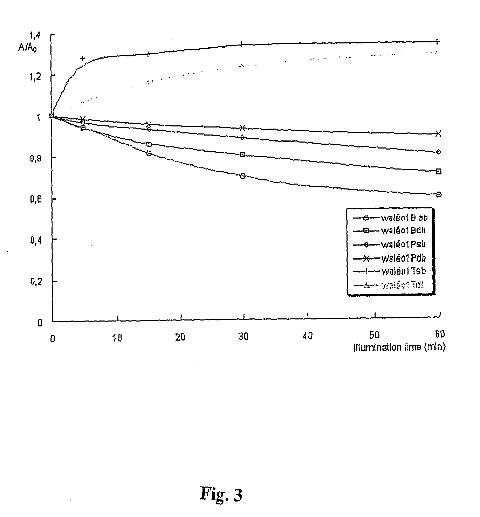

During illumination, the absorption spectrum of Waleo1P single stranded and Waleo1P double stranded (ds) undergoes a hypochromic effect around 450 nm (MLCT absorption band) and a hyperchromic effect above 500 nm whereas the emission drops during the illumination, which is known to be characteristic of a photodechelation of the Ru(II) complex (Fig.2). In contrast, the absorption spectra of WaleolT ss shows a hyperchromic and hypsochromic effect of the MLCT absorption band around 420 nm. This is characteristic of the formation of a photoadduct; that means that the Ru(Tap)₂Phen²⁺ complex anchored on the ODN reacts, under illumination, with a G base. The origin of the photo-product can be double: either it is an intramolecular photo-product (the Ru(Tap)₂Phen²⁺ complex in its excited state reacts with the guanine of its own ODN strand) or it is an intermolecular photoadduct (the Ru(Tap)₂Phen²⁺ complex in its excited state reacts with the guanine of another Ru-ODN). The same behaviour is observed with the double stranded Waleo1T sample with the only difference that the photoreaction with the single strand is faster than with the double strand (Fig.3).

The results obtained with the 5'- modified ODNs are very similar to those described for the 3' - modified ODNs. The only difference is in the emission spectrum evolution of Waleo2Tds compared to Waleo1Tds. Waleo2Tds shows very poor emission compared to Waleo1Tds and its luminescence does not evolve during illumination. This may result from the direct vicinity of 3 Gs on the extremity of the 3' end of the target strand. The presence of 3 guanines might favour the electron transfer between one of them and the excited ruthenium complex and thereby quenches the luminescence of its excited state.

To check the nature of the photoadduct obtained by illuminating WaleolT alone and in presence of its complementary strand, polyacrylamide gel electrophoresis experiments were performed.

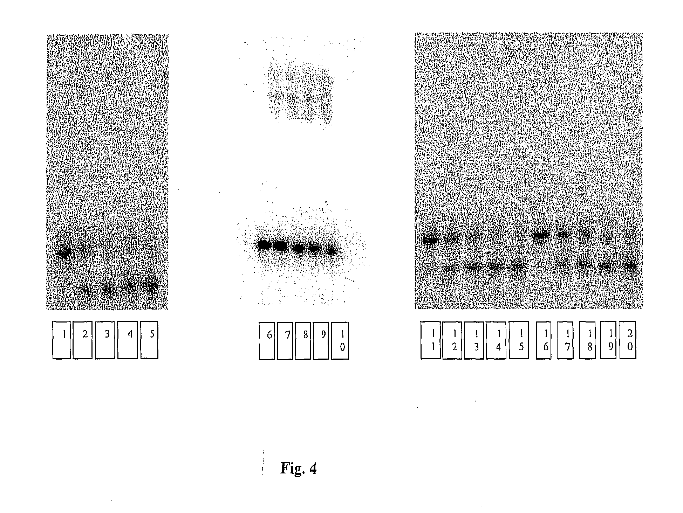

Therefore, 5'-³²P labeled Waleo1T and Waleo1Tds solutions were illuminated with a monochromatic laser (λ = 452 nm) during 5, 10, 15 and 30 min (Fig 4). Lane 1 is the native Waleo1T Ru-ODN. During illumination, Waleo1T is consumed and a new band appears migrating faster than the starting material (lanes 2 to 5). This observation is consistent with the hypothesis of intramolecular photoreaction of Waleo1T. After 30 min of illumination, 85 % of intramolecular photoproduct were obtained.

When WaleolT is hybridized with its complementary strand and illuminated as described, a new band appears on the electrophoresis gel, migrating much slower than native Waleo1T, consistent with a photocrosslinking product (lane 7 to 10). The amount of photocrosslinking is around 40 % of the starting material after 30 min of illumination. On the other hand, no trace of the intramolecular photoproduct can be seen. Thus, Waleo1T under illumination undergoes an intramolecular reaction whereas the double strand leads selectively to the photocrosslinking product.

In a second step, the complementary strand (Waleo3) were replaced by another ODN strand containing one or more guanine bases in order to check the specificity of the photoactive molecule. Two scramble sequences SC1: 5'-TTT TCG TTT TAA ATT AT-3' with 1 G and SC2: 5'-TAA ATT TAA GGA AAA AA-3' with 2 Gs were used. After illumination, PAGE experiments showed absolutely no photocrosslinking product, but only intramolecular photoproduct could be detected (Lane 11 to 20).

In conclusion, Waleo1T photoreacts very specifically with its target sequence and leaves all other guanines containing oligonucleotides intact. In the presence of any other sequence than the targeted sequence, Waleo1T undergoes an intramolecular reaction rather than reacting with a non-recognized guanine. In other words, if the photo-active molecule does not find its target (its complementary strand), it kills itself and does not damage any other DNA sequence.

## Claims

1. A oligonucleotide comprising a photoreactive Ru (II) complex anchored on the oligonucleotide, **characterised in that** the oligonucleotide comprises one or more guanine unit(s).

2. The oligonucleotide according to claim 1, wherein the photoreactive Ru (II) complex is anchored upon the oligonucleotide sequence by a chemical linker.

3. The oligonucleotide according to the claim 1 or 2, which is an antisense oligonucleotide.

4. A diagnostic kit comprising the oligonucleotide according to claims 1 to 3 and possibly a suitable device that emits photons.

5. A pharmaceutical composition comprising an adequate pharmaceutical carrier or diluent, the oligonucleotide according to any of the preceding claims 1 to 3, and a photon.

6. Use of the pharmaceutical composition according to claim 5 for the manufacture of a medicament in the treatment and/or the prevention of an hyper-proliferative disorder.

7. Use according to the claim 6, wherein the hyper-proliferative disorder is a disorder affecting an epithelial cell.

8. Use according to the claim 7, wherein the epithelial cell is a tumor cell, selected from the group consisting of a skin tumoral cell, a digestive track tumoral cell, lung tumoral cell and tumoral cell of the reproductive and/or urinary organ of a mammal, including a human.
